# EUROPEAN PATENT SPECIFICATION

(11) **EP 2 855 693 B1**
(45) Date of publication and mention of the grant of the patent: **11.05.2016**
(21) Application number: 13737399.9
(22) Date of filing: 27.05.2013
(51) Int. Cl.: C12P 33/06

(54) **PROCESS FOR THE SELECTIVE REDUCTION OF BILE ACIDS, THEIR SALTS OR DERIVATIVES, IN A BIPHASIC SYSTEM**
VERFAHREN ZUR SELEKTIVEN REDUKTION VON GALLENSÄUREN, DEREN SALZEN ODER DERIVATEN IN EINEM 2-PHASENSYSTEM
PROCÉDÉ DE RÉDUCTION SÉLECTIVE DES ACIDES BILIAIRES, DE LEURS SELS OU DÉRIVÉS, DANS UN SYSTÈME BIPHASIQUE

(30) Priority: 28.05.2012 IT MI20120918
(43) Date of publication of application: 08.04.2015
(73) Proprietor: Prodotti Chimichi e Alimentari S.P.A., 15060 Basaluzzo (AL) (IT)
(72) Inventor: MONTI, Daniela, I-22020 Faloppio (CO) (IT); FERRANDI, Erica Elisa, I-20861 Brugherio (MB) (IT); RIVA, Sergio, I-20822 Seveso (MB) (IT); POLENTINI, Fausto, I-15067 Novi Ligure (AL) (IT)
(74) Representative: Bottero, Carlo
(86) International application number: PCT/IB2013/054373
(87) International publication number: WO 2013/179210

(56) References cited:
- EP-A1- 1 114 869
- ROBERTO BOVARA ET AL: "Enzymatic .alpha./.beta. inversion of the C-7-hydroxyl of steroids", THE JOURNAL OF ORGANIC CHEMISTRY, vol. 58, no. 2, 1 January 1993 (1993-01-01), pages 499-501, XP055042597, ISSN: 0022-3263, DOI: 10.1021/jo00054a039 cited in the application

## Description

The present invention relates to a new process for the industrial production of ursodeoxycholic acid from different bile acids (shown by way of example in Schema 1), their salts or derivatives, which are easily commercially obtainable, such as for example 7-ketolithocholic acid (3α-hydroxy-7-keto-5β-cholanoic acid, (I)), 7-keto-deoxycholic acid methyl ester (3α,12α-dihydroxy-7-keto-5β-cholanoic acid methyl ester, (II)) and 7,12 diketo-lithocholic acid methyl ester (3α-hydroxy-7,12-diketo-5β-cholanoic acid methyl ester, (III)).

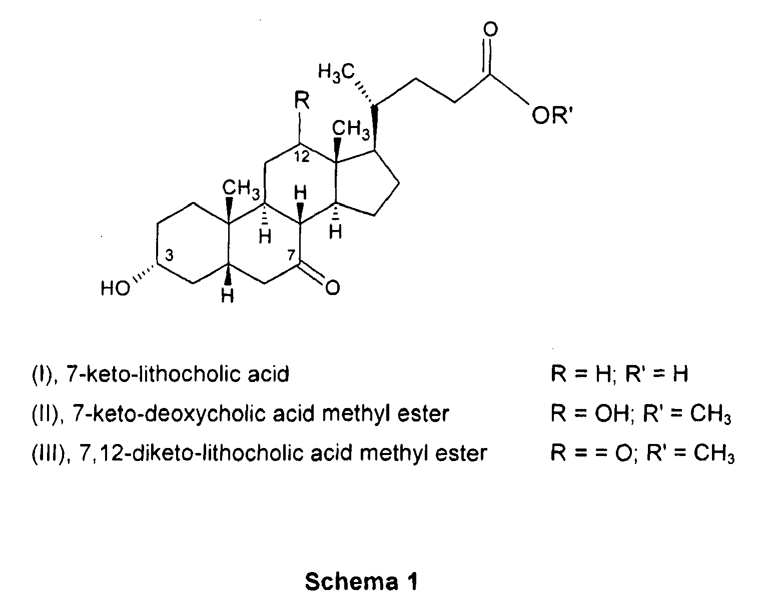

Ursodeoxycholic acid, i.e. 3α,7β-dihydroxy-5β-cholanoic acid (UDCA), of which the formula is presented in the formula (IV) below (Schema 2), 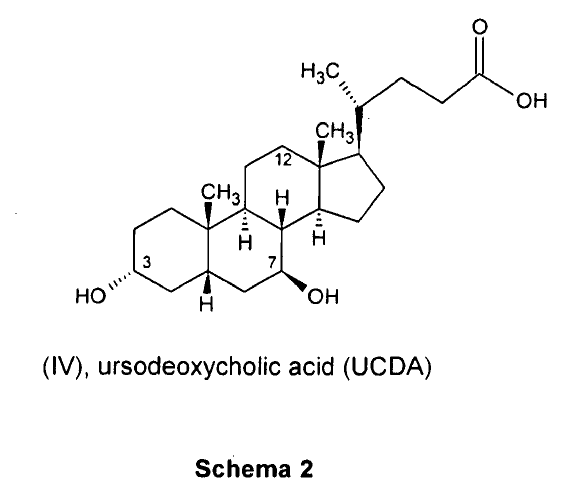 is a compound normally present in small quantities in human bile, where it increases the solubilising capacity of the bile with respect to cholesterol. UDCA is known for its therapeutic properties: it is indeed used for the treatment of dysfunctions of the liver region, including the dissolution of cholesterol gallstones, primitive biliary cirrhosis and sclerosing cholangitis.

It is therefore of great interest to provide a process for the industrial production of UDCA.

Currently, UDCA is produced via chemical synthesis from cholic acid (3α,7α,12α-trihydroxy-5β-cholanoic acid (CA)), extracted from bovine bile, by means of the formation of the intermediate 12-keto-chenodeoxycholic acid (Hoffmann A. F., Acta Chem. Scand., 1963, 17, 173-186; Sammuelson B., Acta Chem. Scand., 1960, 14, 17-20).

Various synthetic strategies instead of the traditional chemical syntheses of ursodeoxycholic acid have been proposed, for example as described in Bovara R., Carrea G., Riva S. and Secundo F. Biotechnology letters 1996, 18, 305-308.

These strategies use different oxidised derivatives of cholic acid or of chenodeoxycholic acid (3α,7α-dihydroxy-5β-cholanoicacid (CDCA)), such as for example 7-keto-lithocholic acid, 7-keto-deoxycholic acid, and 7,12-diketo-lithocholic acid, which are reduced in position 7 either chemically or enzymatically.

The chemical procedures generally result in the reduction of the ketone in position 7 of the preselected bile acid by means of metal sodium/alcohol (for example methanol, isopropanol) (Sammuelson B., already cited), however the yields and the conversions obtained by means of such a method are not completely satisfactory in terms of stereoselectivity, leading to the formation of mixtures of UDCA and CDA (usually in a ratio around 85:15).

Alternative synthetic methods proposed include the reduction of the ketone in position 7 of 7-keto-lithocholic acid by means of potassium/tert-amyl alcohol (Batta A.K. et al., J. Lipid Res., 1991, 32, 977-983) or by means of electrochemical reduction (Zhao H. Tian H. et al.,J. Appl. Electrochem., 2010, 40, 1307-1316), however none of these methods is currently used on an industrial scale.

As an alternative to chemical synthesis, the reduction of the ketone in position 7 of the bile acid can be achieved enzymatically using NAD(P)H-dependent hydroxysteroid dehydrogenase (HDSH) activities coupled with suitable regeneration systems of the cofactor, which can therefore be reacted in catalytic and non-stoichiometric quantities.

The HSDH activities demonstrate an almost absolute regioselectivity and stereoselectivity, and can therefore be used to catalyse reactions without resulting in preventive protection of the hydroxyl groups not requiring oxidation/reduction, conversely to that which occurs in chemical oxidations/reductions.

Since the catalysed reactions of oxidation-reduction from HSDHs are reversible, the regeneration system of the cofactor coupled with the reduction reaction can be utilised not only for the purpose of reducing the quantity of NAD(P)H to be used, but also to promote the equilibrium of the reaction towards increased percentages of conversion (Kroutil W. et al.,Adv. Synth. Catal., 2004, 346, 125-142).

Quantitative conversions can be obtained by coupling the reduction reaction with a non-reversible oxidation reaction of a co-substrate catalysed by a second dehydrogenase. The most commonly used regeneration methods include the oxidation of ammonium formate to give carbonic anhydride catalysed by formate dehydrogenase (FDH) or the oxidation of glucose to give glucono-δ-lactone, which spontaneously hydrolyses to give gluconic acid, catalysed by glucose dehydrogenase (GDH). The second method is generally used more often than the first since the GDHs can be obtained with optimum yields in terms of activity and are even stable in conditions of industrial use, whereas the FDHs currently available are characterised by limited stability and specific activity (van der Donk W. , Zhao H. Curr. Opin. Biotech., 2003, 14, 421-426). The literature reports numerous examples of reduction reactions of ketones coupled with the glucose/GDH regeneration system, as described in Kosjek B, et al., Org. Process Res. Dev., 2008,12, 584-588; Eguchi T.et al., Biosci. Biotechnol. Biochem., 1992, 56, 701-703;and Nidetzky B. et al., Biotechnol. Bioeng. 1996, 52, 387-396.

The glucose/GDH system has therefore been used in the industrial production of pharmaceuticals, for example testosterone and L-carnitine, as reported in patents MI2009A001168 and US4542098 respectively.

With regard to the reduction reactions of bile acids, some examples coupled with the glucose/GDH system for the regeneration of the NAD(P)H cofactor are described in Riva S. et al., J. Org. Chem., 1986, 51, 2902-2906; Bovara R. et al., Biotechnology letters, already cited; Carrea G.et al., Biotechnol. Lett.,1992, 12, 1131-1135; Bovara R. et al., and J. Org. Chem., 1993, 58, 499-501.

Such reactions were performed on laboratory scale however and only at low concentrations of the bile acid substrate (usually 12.5 mM, equal to 5 g/l).

In addition, one disadvantage observed when using 7-keto-lithocholic acid as substrate in the stereoselective and regioselective reduction reaction catalysed by a 7β-HSDH to give the product of interest, UDCA, is the fact that, even at substrate concentrations equal to 50 mM (20 g/l), the reaction mixture will be subject to complete gelification, thus impeding the progression of the reaction itself until completion.

The formation of the gel seems to be caused both by a natural tendency of the bile acids, and in particular of UDCA, to form supramolecular structures and by the presence in the reaction environment of the glucose/gluconic acid co-substrates/co-products, which, due to their pronounced hydrophilicity, tend to remove water molecules from the other solutes present (in this case the bile acids) and therefore tend to promote structural aggregation.

However, with the known reaction conditions, it is not possible to implement industrial production of UDCA, which, in order to be considered acceptable from an economical viewpoint, has to be performed at concentrations of starting substrate at least equal to 50 - 100 mM (20-40 g/l).

The document already cited above Bovara R. et al., J. Org. Chem. describes the reduction reaction of 7-keto-deoxycholic acid methyl ester (25 mM in organic phase, 10 g/l) catalysed by a 7β-HSDH in a phosphate buffer biphasic system: ethyl acetate =1.5 : 1 coupled with the glucose/GDH system for the regeneration of the NAD(P)H cofactor, but the conversions obtained here are less than 20% and therefore are not applicable at industrial level, presumably due to enzymatic inactivation.

In addition, it is of interest to note how the bile acids are soluble in water only in the form of the corresponding sodium or potassium salts at pH greater than 7.5-8.0, whereas, in an organic solvent, they can be soluble in the form of acids (for example 7-keto-lithocholic acid) or in the form of methyl esters (for example 7-keto-deoxycholic acid methyl ester). For this reason, in a biphasic system, the quantity of bile acid or of a derivative thereof present in the aqueous phase isvery low. Consequently, the reactions catalysed by HSDH in a biphasic system can be quicker and more quantitative than those in homogeneous aqueous phase since it has been observed that the HSDHs are inhibited at elevated concentrations (> 50 mM) of bile acids (Ferrandi E.E., Bertolesi G.M., Polentini F., Negri A., Riva S., Monti D. Appl. Microbiol. Biotechnol., 2012, in press, DOI: 10.1007/s00253-011-3798-x).

Now, a new process has surprisingly been found for the regioselective enzymatic reduction of bile acids, their salts or derivatives, such as 7-keto-lithocholic acid, 7-keto-deoxylithocholic acid methyl ester and 7,12-diketo-lithocholic acid methyl ester, which, wherein said process uses NAD(P)H-dependent 7β-hydroxysteroid dehydrogenase (7β-HSDH) in the presence of a glucose dehydrogenase (GDH) in biphasic systems, thus making possible to dissolve the starting substrates in elevated quantities, avoid the formation of gel, avoid enzymatic inactivation, and obtain quantitative conversions.

The process of the invention was discovered by confirming experimentally the influence of various parameters, such as the quantity of HSDH and GDH enzymes, the concentration of the bile acid substrates and of the glucose co-substrate, temperature and pH, on the degree of conversion of the reduction reaction in the presence of various organic solvents.

The object of the present invention is therefore a process for the regioselective enzymatic reduction in position 7 of bile acids, their salts or derivatives, such as 7-keto-lithocholic acid, 7-keto-deoxycholic acid methyl ester and 7,12-diketo-lithocholic acid methyl ester in a biphasic system, which comprises reacting said compounds in the presence of a NAD(P)H-dependent 7β-hydroxysteroid dehydrogenase (7β-HSDH), of a regeneration system of the NAD(P)H cofactor comprising glucose as co-substrate, of a glucose dehydrogenase (GDH) and an organic solvent selected from the group consisting of linear or branched C₄-C₁₀ alkyl alcohols, linear or branched C₄-C₁₀ ketones and linear or branched C₃-C₅ esters of acetic acid.

The alcohols 2-hexanol, 3-octanol and 4-methyl-2-pentanol, the ketone solvent 2-pentanone and the ester i-propylacetate are particularly preferred.

The NAD(P)H-dependent 7β-HSDHs used in the process of the present invention preferably have an enzymatic activity ranging from 30 to 50 U/ml, where enzymatic activity (U) means the quantity of 7β-HSDH enzyme able to transform, within a minute, a micromole of the 7-keto-lithocholic acid substrate into the corresponding product reduced in position 7 in an assay solution containing 12.5 mM of 7-keto-lithocholic acid.

The NAD(P)H-dependent GDHs used in the process of the present invention preferably have an enzymatic activity ranging from 20 to 30 U/ml, where enzymatic activity (U) means the quantity of GDH enzyme able to transform, within a minute, a micromole of the glucose substrate into the corresponding oxidised glucono-δ-lactone product in an assay solution containing 50 mM of glucose.

The starting compounds, i.e. the substrates of formula (I), (II) or (III), are preferably used at a concentration ranging from 1% and 12% (weight/volume) in an organic phase (25-300 mM), and more preferably are used at a concentration ranging from 5% to 10% (weight/volume) (125-250 mM).

The process of the present invention is preferably carried out at room temperature, i.e. at about 20° C-25°C and is preferably carried out at a pH of 7-7.5.

The buffer used is preferably the buffer potassium phosphate.

The duration of the process described here is preferably between 4 hours and 24 hours, and is more preferably between 5 and 10 hours.

The NAD(P)⁺ cofactor is preferably used in catalytic quantities at concentrations ranging from 100 and 1000 µM, preferably between 300 and 500 µM.

The glucose co-substrate is used at equimolar concentrations with respect to the starting substrate, but preferably in slight excess (preferably between 20 and 50%).

In accordance with one aspect of the present invention, the reduction reaction is carried out in a biphasic system by means of the addition of an aqueous phase containing a NAD(P)H-dependent 7β**-**hydroxysteroid dehydrogenase (7β-HSDH), such as the 7β-HSDH that catalyses the reduction reaction of the bile acid, indicated in Schema 3, a glucose dehydrogenase that regenerates the NAD(P)H cofactor, glucose as co-substrate of the regeneration reaction, and the NAD(P)⁺ cofactor in catalytic quantities to an organic phase selected from those described above, in which the bile acid of interest is dissolved, such as the compound (I), (II) or (III) as indicated in Schema 3.

In Schema 3, the compound (IV) is UDCA, formed in the reduction process illustrated by way of example at point a) starting from the starting compound of formula (I), whereas point b) describes the oxidation reaction of the glucose coupled with the reduction reaction referred to at point a). The products of the reduction reactions of the compounds of formulas (II) and (III) are, respectively, ursocholic acid methyl ester (3α,12α,7β-trihydroxy-5β-cholanoic acid methyl ester, (V)) and 12-keto-ursodeoxycholic acid methyl ester (3α,7β-dihydroxy-12-keto-5β-cholanoic acid methyl ester, (VI)).

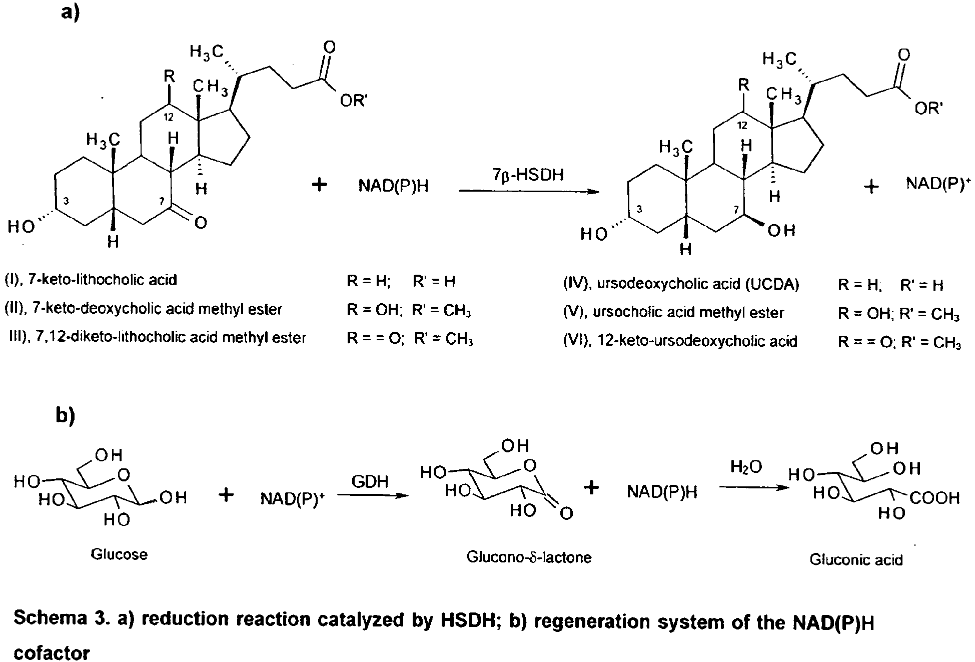

The 7β-HSDH used in the process of the invention can be the NADPH-dependent 7β-HSDH from *Clostridium absonum* described in Ferrandi E.E. et *al.* already cited above, the NADPH-dependent 7β-HSDH from *Collinsella aerofaciens* described in Liu L. et al., Appl. Microbiol. Biotechnol., 2011, 90, 127-35, or the NADPH-dependent 7β-HSDH from *Xanthomonas maltophilia* described in Pedrini P. et al., Steroids, 2006, 71, 189-198.

The glucose dehydrogenase used in the process of the invention may be that from *Bacillus megaterium* (Heilmann H. J.et al., Eur. J. Biochem., 1988, 174, 485-490; Ebeling W et al., US5250415), from *Bacillus subtilis* (Fujita Y.et al., J. Bacteriol., 1977, 132, 282-293), from *Gluconobacter suboxydans* (Adachi O. et al., Agric. Biol. Chem., 1980, 44, 301-308), from *Thermoplasma acidophilum* (Smith L. D. et al., Biochem. J., 1989, 261, 973-977; Bright J. R. et al., Eur. J. Biochem., 1993, 211, 549-554),from *Sulfolobus solfataricus* (Giardina P. et al., Biochem. J., 1986, 239, 517-522), from *Pseudomonas* (Sogabe A. et al. US5298411), or from *Aspergillus oryzae* (Tsuji Y. et al., US7655130).

The derivatives of formulas (V) and (VI) obtained with the process of the present invention when the starting substrates are 7-keto-deoxycholic acid methyl ester (II) or 7,12-diketo-lithocholic acid methyl ester (III) can be used in the chemical synthesis of UDCA, as described in patent EP0072293 and in Hoffmann A. F., or Sammuelson B. (already cited).

The examples below are to be considered as nonlimiting illustrations of the invention.

### EXAMPLES

### Example 1-Enzymatic reduction in a biphasic system of 7-keto-lithocholic acid (I)

4 g (10 mmol) of 7-keto-lithocholic acid (I) were dissolved in 40 ml of 4-methyl-2-pentanolo (10% weight/volume, 250 mM) in the presence of 4 ml of deionised water by heating at a temperature of 90°C for a few minutes. The solution was left to cool to about 23° C, and 8 ml of phosphate buffer 100 mM pH 9.2 and 2.2 g (12.2 mmol) of glucose were added. The system was kept under stirring in order to mix the two phases, and 15 mg (0.023 mmol) of NAD⁺, 130 U of 7β-HSDH (3.2 ml of a solution containing 40 U/ml of 7β-HSDH) and 80 U of GDH (3.2 ml of a solution containing 25 U/ml of GDH) were then added. The temperature and the pH were kept constant during the course of the reaction. Complete conversion (> 99.5%) of the 7-keto-lithocholic acid (I) into ursodeoxycholic acid (IV) was achieved in a time of 5 h. The degree of conversion was evaluated by means of HPLC analysis.

By way of comparison, the best conditions for the reduction reaction of the substrate (I) to the product (IV) in homogeneous aqueous phase (monophasic) will be reported below.

The reaction was carried out at 20° C and pH 8.0 in a total volume of 100 ml, containing: 4 g (10 mmol) of the sodium salt of 7-keto-lithocholic acid (I) at pH 8.0 (4% weight/volume, 100 mM, obtained by diluting an aqueous solution of 0.2 M of the sodium salt of the 7-keto-lithocholic acid at pH 8.0), 2.2 g (12.2 mmol) of glucose, 240 U of 7β-HSDH (6 ml of a solution containing 40 U/ml of 7β-HSDH) and 150 U of GDH (6 ml of a solution containing 25 U/ml of GDH), 40 mg (0.06 mmol) of NAD⁺, 50 mM of potassium phosphate buffer, pH 8.0.

In a time of about 16 h, a conversion > 99% of the 7-keto-lithocholic acid (I) into ursodeoxycholic acid (UDCA (IV)) was observed, however, even under stirring, the solution gelifies, impeding completion of the reaction.

### Example 2 -Enzymatic reduction in biphasic system of 7-keto-deoxycholic acid methyl ester (II) or of 7,12-diketo-lithocholic acid methyl ester (III)

0.4 g (1 mmol) of 7-keto-deoxycholic acid methyl ester (II) was dissolved in 5 ml of i-propylacetate (8% weight/volume, 200 mM). 3.5 ml of phosphate buffer 50 mM pH 8.0 and 220 mg (1.22 mmol) of glucose were then added. The system was kept under stirring in order to mix the two phases, then 0.2 ml of a solution of 6 mg/ml of NAD⁺ (0.0018 mmol), 20 U of 7β-HSDH (0.5 ml of a solution containing 40 U/ml of 7β-HSDH) and 7.5 U of GDH (0.3 ml of a solution containing 25 U/ml of GDH) were then added. The temperature and the pH were kept constant over the course of the reaction. Complete conversion (> 99.5%) of the 7-keto-deoxycholic acid methyl ester (II) into an ursocholic acid methyl ester (V) was achieved in a time of 5 h. The degree of conversion was evaluated by means of HPLC analysis. The same reaction conditions were applied for the reduction reaction of the 7,12-diketo-lithocholic acid methyl ester (III) to give the 12-keto-ursodeoxycholic acid methyl ester (VI). Complete conversion (> 99.5%) of the starting substrate into the expected product was achieved in a time of 5 h.

## Claims

1. A process for the regioselective enzymatic reduction in position 7 of compounds having formula 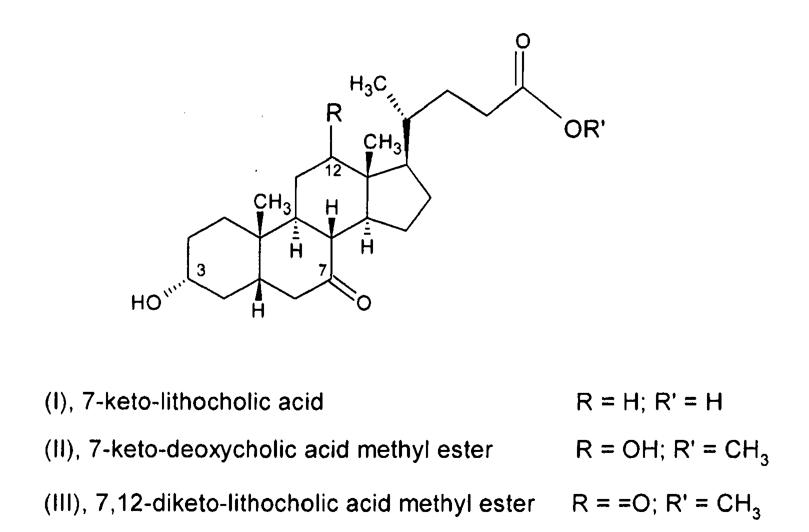 or their salts or derivatives, which comprises reacting said compounds in the presence of a NAD(P)H-dependent 7β-hydroxysteroid dehydrogenase (7β-HSDH), of a regeneration system of the NAD(P)H cofactor comprising glucose as co-substrate, a glucose dehydrogenase (GDH) and an organic solvent selected from the group consisting of linear or branched C₄-C₁₀ alkyl alcohols, linear or branched C₄-C₁₀ ketones and linear or branched C₃-C₅ esters of acetic acid.

2. The process according to claim 1, wherein the organic solvent is 2-hexanol, 3-octanol, 4-methyl-2-pentanol, 2-pentanone or i-propylacetate ester.

3. The process according to claim 1, wherein the NAD(P)H-dependent 7β-HSDH has an enzymatic activity ranging from 30 to 50 U/ml.

4. The process according to claim 1, wherein the NAD(P)H-dependent GDH has an enzymatic activity ranging from 20 to 30 U/ml.

5. The process according to claim 1, wherein the compounds having formula (I), (II) or (III), their salts or derivatives, have a concentration ranging from 1% to 12% (weight/volume) in an organic phase (25-300 mM), and more preferably ranging from 5% to 10% (weight/volume) (125-250 mM).

6. The process according to any one of the previous claims, wherein the NAD(P)⁺ cofactor is used in catalytic quantities at concentrations ranging from 100 to 1000 µM, preferably from 300 to 500 µM.

7. The process according to any one of the previous claims, wherein the glucose co-substrate is used at equimolar concentrations, or in slight excess, preferably ranging from 20 to 50%, with respect to each of the compounds (I), (II) or (III).

8. The process according to any one of the previous claims, wherein the reduction is quantitative, or higher than or equal to 99.5%.

9. The process according to any one of the previous claims, carried out at room temperature, i.e. at about 20°C - 25°C.

10. The process according to any one of the previous claims, carried out at a pH ranging from 7 to 7.5.

11. The process according to claim 10, also comprising a buffer system, preferably consisting of the buffer potassium phosphate.

12. A process for the preparation of ursodeoxycholic acid (IV), **characterized in that** it comprises a process according to any one of the claims from 1 to 11.

13. A process for the preparation of the methyl ester of ursocholic acid (V), **characterized in that** it comprises a process according to any one of the claims from 1 to 11.

14. A process for the preparation of the methyl ester of 12-keto-ursodeoxycholic acid (VI), **characterized in that** it comprises a process according to any one of the claims from 1 to 11.

## Patentansprüche

1. Verfahren zur regioselektiven, enzymatischen Reduktion in Position 7 von Verbindungen mit der Formel 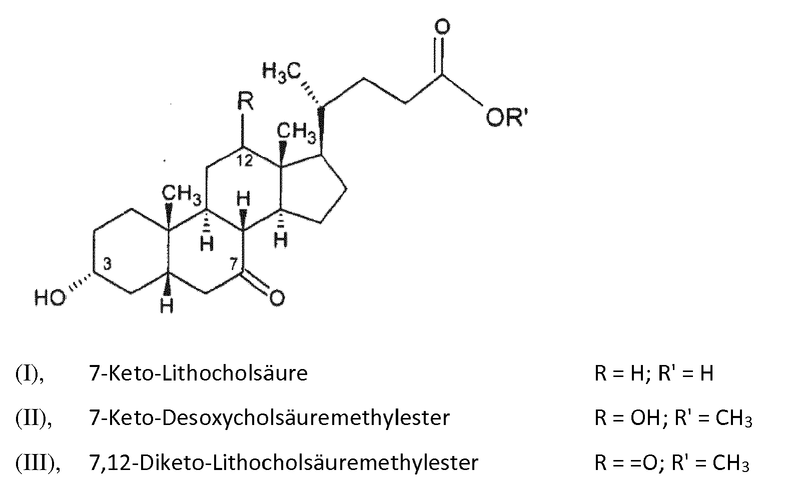 oder deren Salzen oder Derivaten, welches das Umsetzen der Verbindungen in Gegenwart einer NAD(P)H-abhängigen 7β-Hydroxysteroiddehydrogenase (7β-HSDH), eines Regenerationssystems des NAD(P)H Cofactors umfasst, umfassend Glucose als Co-Substrat, eine Glucose-Dehydrogenase (GDH) und ein organisches Lösemittel, ausgewählt aus der Gruppe bestehend aus linearen oder verzweigten C₄-C₁₀ Alkylalkoholen, linearen oder verzweigten C₄-C₁₀ Ketonen und linearen oder verzweigten C₃-C₅ Estern der Essigsäure.

2. Verfahren nach Anspruch 1, wobei das organische Lösemittel 2-Hexanol, 3-Octanol, 4-Methyl-2-Pentanol, 2-Pentanon oder i-Propylacetatester ist.

3. Verfahren nach Anspruch 1, wobei die NAD(P)H-abhängige 7β-HSDH eine Enzymaktivität im Bereich von 30 bis 50 U/ml aufweist.

4. Verfahren nach Anspruch 1, wobei die NAD(P)H-abhängige GDH eine Enzymaktivität im Bereich von 20 bis 30 U/ml aufweist.

5. Verfahren nach Anspruch 1, wobei die Verbindungen mit den Formeln (I), (II) oder (III), deren Salze oder Derivate eine Konzentration im Bereich von 1 % bis 12 % (Gewicht/Volumen) in einer organischen Phase (25 - 300 mM), und besonders bevorzugt im Bereich von 5 % bis 10 % (Gewicht/Volumen) (125 - 250 mM) aufweisen.

6. Verfahren nach einem der vorstehenden Ansprüche, wobei der NAD(P)⁺ Cofaktor in katalytischen Mengen in Konzentrationen im Bereich von 100 bis 1000 µM, vorzugsweise von 300 bis 500 µM verwendet wird.

7. Verfahren nach einem der vorstehenden Ansprüche, wobei das Glucose-Co-Substrat in äquimolaren Konzentrationen oder in geringem Überschuss, vorzugsweise im Bereich von 20 bis 50 % bezogen auf die jeweiligen Verbindungen (I), (II) oder (III) verwendet wird.

8. Verfahren nach einem der vorstehenden Ansprüche, wobei die Reduktion quantitativ oder größer oder gleich 99,5 % ist.

9. Verfahren nach einem der vorstehenden Ansprüche, durchgeführt bei Raumtemperatur, d.h. bei ca. 20 °C - 25 °C.

10. Verfahren nach einem der vorstehenden Ansprüche, durchgeführt bei einem pH-Wert im Bereich von 7 bis 7,5.

11. Verfahren nach Anspruch 10, ferner umfassend ein Puffersystem, vorzugsweise bestehend aus Kaliumphosphatpuffer.

12. Verfahren zur Herstellung von Ursodesoxycholsäure (IV), **dadurch gekennzeichnet, dass** es ein Verfahren nach einem der Ansprüche 1 bis 11 umfasst.

13. Verfahren zur Herstellung des Methylesters von Ursodesoxycholsäure (V), **dadurch gekennzeichnet, dass** es ein Verfahren nach einem der Ansprüche 1 bis 11 umfasst.

14. Verfahren zur Herstellung des Methylesters von 12-Keto-Ursodesoxycholsäure (VI), **dadurch gekennzeichnet, dass** es ein Verfahren nach einem der Ansprüche 1 bis 11 umfasst.

## Revendications

1. Procédé de réduction enzymatique régiosélective dans la position 7 de composés ayant la formule : 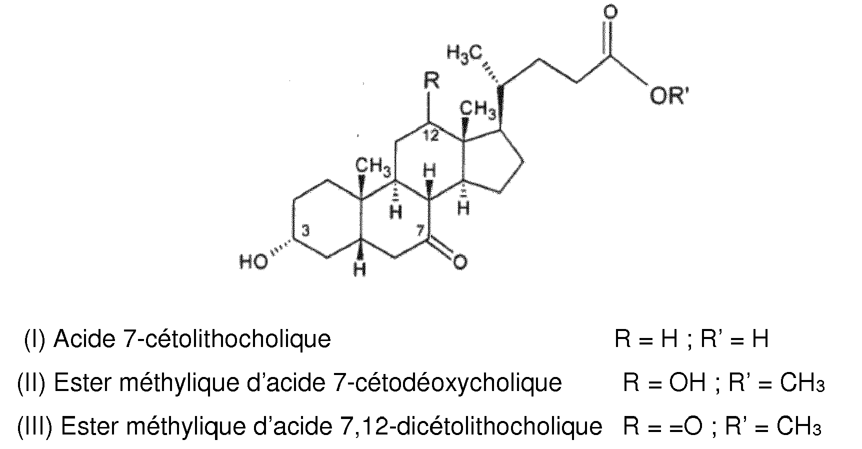 ou leurs sels ou dérivés, qui comprend la réaction desdits composés en présence d'une 7β-hydroxystéroïde déshydrogénase (7β-HSDH) dépendant de NAD(P)H, d'un système de régénération du cofacteur NAD(P)H comprenant du glucose comme co-substrat, une glucose déshydrogénase (GDH) et un solvant organique sélectionné à partir du groupe comprenant des alcools d'alkyle C₄-C₁₀ linéaires ou ramifiés, des cétones C₄-C₁₀ linéaires ou ramifiées et des esters d'acide acétique C₃-C₅ linéaires ou ramifiés.

2. Procédé selon la revendication 1, dans lequel le solvant organique est 2-hexanol, 3-octanol, 4-méthyle-2-pentanol, 2-pentanone o ester acétate d'i-propyle.

3. Procédé selon la revendication 1, dans lequel le 7β-HSDH dépendant de NAD(P)H a une activité enzymatique dans la plage de 30 à 50 U/ml.

4. Procédé selon la revendication 1, dans lequel la GDH dépendant de NAD(P)H a une activité enzymatique dans la plage de 20 à 30 U/ml.

5. Procédé selon la revendication 1, dans lequel les composés ayant la formule (I), (II) ou (III), leurs sels ou dérivés, ont une concentration dans la plage de 1% à 12% (poids/volume) dans une phase organique (25-300 mM), et de manière davantage préférée dans la plage de 5% à 10% (poids/volume) (125-250 mM).

6. Procédé selon l'une quelconque des revendications précédentes, dans lequel le cofacteur NAD(P)* est utilisé en quantités catalytiques à des concentrations dans la plage de 100 µM à 1000 µM, de préférence de 300 à 500 µM.

7. Procédé selon l'une quelconque des revendications précédentes, dans lequel le co-substrat de glucose est utilisé à des concentrations équimolaires, ou légèrement en excès, de préférence dans la plage de 20 à 50%, par rapport à chacun des composés (I), (II) ou (III).

8. Procédé selon l'une quelconque des revendications précédentes, dans lequel la réduction est quantitative, ou supérieure ou égale à 99,5%.

9. Procédé selon l'une quelconque des revendications précédentes, exécuté à température ambiante, c'est-à-dire à environ 20°C - 25°C.

10. Procédé selon l'une quelconque des revendications précédentes, exécuté à un pH dans la plage de 7 à 7,5.

11. Procédé selon la revendication 10, comprenant également un système tampon, comprenant de préférence un tampon de phosphate de potassium.

12. Procédé pour la préparation d'acide ursodéoxycholique (IV), **caractérisé en ce qu'**il comprend un procédé selon l'une quelconque des revendications 1 à 11.

13. Procédé pour la préparation de l'ester méthylique d'acide ursocholique (V), **caractérisé en ce qu'**il comprend un procédé selon l'une quelconque des revendications 1 à 11.

14. Procédé pour la préparation de l'ester méthylique d'acide 12-céto-ursodéoxycholique (VI), **caractérisé en ce qu'**il comprend un procédé selon l'une quelconque des revendications 1 à 11.
